# EUROPEAN PATENT SPECIFICATION

(11) **EP 1 429 600 B1**
(45) Date of publication and mention of the grant of the patent: **31.12.2008**
(21) Application number: 02769997.4
(22) Date of filing: 24.09.2002
(51) Int. Cl.: A01K 67/033

(54) **AN INVERTEBRATE ANIMAL MODEL WITH ALZHEIMER-LIKE PATHOLOGY FOR SCREENING AND TESTING MOLECULES**
INVERTEBRATEN TIERMODELL MIT ALZHEIMER-ÄHNLICHER PATHOLOGIE ZUM SCREENEN UND TESTEN VON MOLEKÜLEN
MODELE D'ANIMAL INVERTEBRE ATTEINT D'UNE PATHOLOGIE DE TYPE ALZHEIMER, DESTINE AU CRIBLAGE ET A L'ANALYSE DE MOLECULES

(30) Priority: 24.09.2001 EP 01122869
(43) Date of publication of application: 23.06.2004
(73) Proprietor: Evotec AG, 22525 Hamburg (DE)
(72) Inventor: REIFEGERSTE, Rita, 20357 Hamburg (DE); GREEVE, Isabell, CH-3032 Hinterkappelen (CH); NITSCH, Roger, M., CH-8702 Zollikon (CH); POHLNER, Johannes, 22175 Hamburg (DE)
(74) Representative: Meyers, Hans-Wilhelm
(86) International application number: PCT/EP2002/010706
(87) International publication number: WO 2003/028446

(56) References cited:
- WO-A-00/24880
- FOSSGREEN A ET AL: "TRANSGENIC DROSOPHILA EXPRESSING HUMAN AMYLOID PRECURSOR PROTEIN SHOW GAMMA-SECRETASE ACTIVITY AND A BLISTERED-WING PHENOTYPE" PROCEEDINGS OF THE NATIONAL ACADEMY OF SCIENCES OF USA, NATIONAL ACADEMY OF SCIENCE. WASHINGTON, US, vol. 95, no. 23, 10 November 1998 (1998-11-10), pages 13703-13708, XP001026070 ISSN: 0027-8424
- BROCQUE LE D ET AL: "PROCESSING OF ALZHEIMER'S DISEASE AMYLOID PRECURSOR PROTEIN IN PICHIA PASTORIS: IMMUNODETECTION OF ALPHA-, BETA- AND GAMMA-SECRETASE PRODUCTS" BIOCHEMISTRY, AMERICAN CHEMICAL SOCIETY. EASTON, PA, US, vol. 37, 1998, pages 14958-14965, XP002184013 ISSN: 0006-2960
- MALLENDER W D ET AL: "CHARACTERIZATION OF RECOMBINANT, SOLUBLE BETA-SECRETASE FROM AN INSECT CELL EXPRESSION SYSTEM" MOLECULAR PHARMACOLOGY, BALTIMORE, MD, US, vol. 59, no. 3, March 2001 (2001-03), pages 619-626, XP001037766 ISSN: 0026-895X
- CHAN H ET AL: "DROSOPHILA MODELS OF HUMAN NEURODEGENERATIVE DISEASE" CELL DEATH AND DIFFERENTIATION, EDWARD ARNOLD, OXFORD, GB, vol. 7, no. 11, November 2000 (2000-11), pages 1075-1080, XP001022997 ISSN: 1350-9047
- YE YIHONG ET AL: "Apoptotic activities of wild-type and Alzheimer's disease-related mutant presenilins in Drosophila melanogaster." JOURNAL OF CELL BIOLOGY, vol. 146, no. 6, pages 1351-1364, XP002226472 ISSN: 0021-9525
- WOLFE MICHAEL S ET AL: "Are presenilins intramembrane-cleaving proteases? Implications for the molecular mechanism of Alzheimer's disease" BIOCHEMISTRY, AMERICAN CHEMICAL SOCIETY. EASTON, PA, US, vol. 38, no. 35, 31 August 1999 (1999-08-31), pages 11223-11230, XP002186847 ISSN: 0006-2960
- VASSAR R ET AL: "BETA-SECRETASE CLEAVAGE OF ALZHEIMER'S AMYLOID PRECURSOR PROTEIN BYTHE TRANSMEMBRANE ASPARTIC PROTEASE BACE" SCIENCE, AMERICAN ASSOCIATION FOR THE ADVANCEMENT OF SCIENCE,, US, vol. 286, no. 5440, 1999, pages 735-741, XP000914811 ISSN: 0036-8075

## Description

The present invention relates to an insect, preferably a fly model of neurodegenerative disease with Alzheimer's disease-like pathological features. Such an insect model may be useful in screening for and testing of compounds and pharmaceutical agents that modulate plaque-formation related to neurodegenerative disease, particularly Alzheimer's disease.

Neurodegenerative diseases, in particular Alzheimer's disease, have a severely debilitating impact on a patient's life. Furthermore, these diseases constitute an enormous health, social, and economic burden. Alzheimer's disease is the most common age-related neurodegenerative condition affecting about 10 % of the population over 65 years of age and up to 45 % over age 85 (for a recent review see Vickers et al., Progress in Neurobiology 2000, 60:139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brain of individuals suffering from Alzheimer's disease are senile plaques, composed of amyloid-β protein, and profound cytoskeletal changes coinciding with the appearance of abnormal filamentous structures and the formation of neurofibrillary tangles. AD is a progressive disease that is associated with early deficits in memory formation and ultimately leads to the complete erosion of higher cognitive function. Currently, there is no cure for AD, nor is there an effective treatment to halt the progression of AD or even a method to diagnose AD ante-mortem with high probability. Several risk factors have been identified that predispose an individual to develop AD, among them most prominently the epsilon4 allele of apolipoprotein E (ApoE). Although there are rare examples of early-onset AD (familiar AD, FAD) which have been attributed to genetic defects in the genes for APP, presenilin-1, and presenilin-2, the prevalent form of late-onset sporadic AD (LOAD) is of hitherto unknown etiologic origin. The late onset and complex pathogenesis of neurodegenerative disorders pose a formidable challenge to the development of therapeutic and diagnostic agents. Therefore, it is very important to develop suitable animal models of neurodegenerative disease which may be useful in the development of such therapeutic and diagnostic agents.

It is an object of the present invention to provide an animal model useful for the screening and testing of compounds and therapeutic agents and for the identification and validation of novel drug targets of neurodegenerative diseases, particularly Alzheimer's disease. Based on the surprising finding that a transgenic fly expressing the genes coding for human APP, human β-secretase and wildtype and/or mutant forms of the endogenous *presenilin* gene of *Drosophila* displays Alzheimer-like pathological features, the present invention further provides methods and applications useful for the sceening of modifier genes that influence the processing of APP.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. The term "gene" as used in the present specification and in the claims comprises both coding regions (exons) as well as non-coding regions (e.g. non-coding regulatory elements such as promoters or enhancers, introns, leader and trailer sequences). The term "regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or lipidation, or by altered signal peptide cleavage or other types of maturation cleavage. These processes may occur post-translationally. The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of a polypeptide or protein. "Variants" include, for example, proteins with conservative amino acid substitutions in highly conservative regions. The term 'AD' shall mean Alzheimer's disease.

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

In one aspect, the present invention features an insect, preferably a fly that has been modified to express a set of genes, said set comprising a gene coding for a vertebrate amyloid precursor protein (APP), or a fragment, or a derivative or a variant thereof, a gene coding for a vertebrate β-secretase (β-site APP cleaving enzyme).

In a preferred embodiment, the insect also expresses a gene coding for a protein having γ-secretase activity. It is herein preferred that said gene is a gene coding for a presenilin. According to the present invention, the presenilin gene can be a vertebrate or an invertebrate gene. It can be a human gene, in particular presenilin-1 or presenilin-2, or fragments, derivatives or variants thereof, for instance known human mutated versions thereof that have been implicated in the early-onset, familiar form of Alzheimer's disease. However, it might also be preferred that the insect of the instant invention expresses the endogenous gene coding for presenilin, or a fragment, or a derivative, or a variant thereof. In one embodiment of the invention, a gene coding for a protein having γ-secretase activity, in particular a gene coding for presenilin, is over-expressed or mis-expressed. The expression can be ectopic, which means that said genes are expressed in a tissue or cell or at a defined developmental stage of the insect, preferably a fly where they are not normally expressed.

In a preferred embodiment of the invention, the insect, preferably a fly is transgenic for said vertebrate amyloid precursor protein (APP), or a fragment, or a derivative or a variant thereof, and for said gene coding for a vertebrate β-secretase (β-site APP cleaving enzyme). Optionally, the insect, preferably a fly is also transgenic for a presenilin gene. The term "transgenic" as used in the instant invention refers to an invertebrate animal that harbors an extra copy or copies of genetic material incorporated into its genome.

In a particularly preferred embodiment, the insect, preferably a fly of the present invention is an insect, preferably a fly, preferably a fly of the genus *Drosophila,* and most preferably *Drosophila melanogaster.* For the practice of the present invention, conventional molecular biology and recombinant DNA techniques are used which are known to a person skilled in the art. Furthermore, techniques relating to the molecular genetics of *Drosophila* are explained in, for example, Robert DB, Drosophila, A Practical Approach (IRL Press, Washington D.C., 1986).

The expression of the vertebrate amyloid precursor protein (APP), or a fragment, or a derivative or a variant thereof, and of a gene coding for a vertebrate β-secretase (β-site APP cleaving enzyme), and, optionally, of a gene coding for presenilin results in an easily visible and identifiable phenotype in the invertebrate animal. As used herein, a phenotype refers to the observable physical or biochemical characteristics of an insect. An altered phenotype, as a consequence of the expression of the vertebrate amyloid precursor protein (APP), and of a gene coding for a vertebrate β-secretase (β-site APP cleaving enzyme), and, optionally, of a gene coding for presenilin, may be the visible consequence of a disrupted biochemical pathway, in particular the pathway of APP processing. The insect of the present invention is characterized by having a phenotype that comprises the generation of β-amyloid. Furthermore, the insect of the present invention shows, as a result of the expression of the vertebrate amyloid precursor protein (APP) and of a gene coding for a vertebrate β-secretase (β-site APP cleaving enzyme) and, optionally, of a gene coding for presenilin, the following phenotypes, either singly or in combination, but not limiting to: plaque deposition, neurodegeneration, degeneration of photoreceptor cells, degeneration of glia cells, reduced viability of the insect, a wing phenotype, in particular the ectopic wing vein phenotype, as described in the example of the present invention.

The insects of the present invention can be used to identify genes that alter or modify an identifiable phenotype and that potentially act in the same biochemical pathways that are responsible for said identifiable phenotype, in particular the pathway of APP processing. Therefore, in a further aspect, the present invention features a method of screening for modifier genes that influence the processing of APP, said method comprises the following steps: (i) providing an insect, preferably a fly that has been modified to express a set of genes, said set comprising a gene coding for a vertebrate amyloid precursor protein (APP), or a fragment, or derivative, or variant thereof, and a gene coding for a vertebrate β-secretase (β-site APP cleaving enzyme), wherein expression of said set of genes results in an identifiable phenotype, (ii) crossing said insect, preferably a fly with an insect having an alteration in a known or candidate gene other than said set of genes, and (iii) screening progeny of said crosses for insects expressing said set of genes and having said alteration in said known or candidate gene other than said set of genes, wherein a modified identifiable phenotype as compared to controls indicates the presence of a modifier gene that influences the processing of APP. The modified identifiable phenotype can be a quantitative or qualitative change, amelioration, or worsening of said identifiable phenotype. An alteration in a known or candidate gene comprises a mutation in said known or candidate gene, for instance a gain-of-function mutation or a loss-of-function mutation. An alteration can result in the mis-expression, over-expression, or under-expression of said known or candidate gene.

It is preferred to determine a human homolog of the modifier gene which has been identified according to the method of the instant invention. The identification and determination of a human homolog gene can be accomplished by a skilled person, for instance, by performing homology searches in publicly available nucleic acid sequence and protein sequence databases using search algorithms known to one of skill in the art.

The insect according to the present invention is useful as a model system for testing and screening reagents and compounds that modulate APP processing. In particular, the insect according to the present invention is extremely beneficial for the testing and screening of reagents and compounds that modulate β-secretase or γ-secretase.

In one further aspect, the present invention pertains to a method of testing a compound, preferably of screening a plurality of compounds useful for the modulation of APP processing, said method comprises the following steps: (i) providing an insect, preferably a fly that has been modified to express a set of genes, said set comprising a gene coding for a vertebrate amyloid precursor protein (APP), or a fragment, or a derivative, or a variant thereof, and a gene coding for a vertebrate β-secretase (β-site APP cleaving enzyme), wherein expression of said set of genes results in an identifiable phenotype, (ii) contacting said insect, preferably a fly with a compound, preferably with a plurality of compound, (iii) assaying for changes in said identifiable phenotype of said insect of step (i) as compared to an insect of step (i) not having been contacted with said compound or plurality of compounds. For the practice of the present invention, it may be desirable, for instance, to simply supplement the food of larvae or adult flies of the instant invention with a compound, or a plurality of compounds, and assay for changes in the identifiable phenotype. The above screening assay may be particularly useful for the identification of compounds that act as inhibitors of β-secretase. Equally, the above screening assay may also be particularly useful for the identification of compounds that act as inhibitors of γ-secretase. However, compounds that modulate the function of gene expression products of modifier genes and their human homologs, for instance modifier genes identified according to the present invention, can also be assayed and screened.

Compounds identified according to the present invention, having an effect on the processing of APP, may be useful for the treatment, prevention, or amelioration of a neurodegenerative disease, in particular Alzheimer's disease.

One further aspect of the present invention relates to a method for producing a medicament comprising the steps of (i) identifying a compound useful for the modulation of APP processing according to the present invention, and (ii) admixing the compound with a pharmaceutical carrier. A medicament obtainable by such a method, as well as a medicament obtained by such a method, is another feature of the present invention.

Other features and advantages of the present invention will be apparent from the following description of the figures and examples which are illustrative only and not intended to limit the remainder of the disclosure in any way.

### Figure Legends

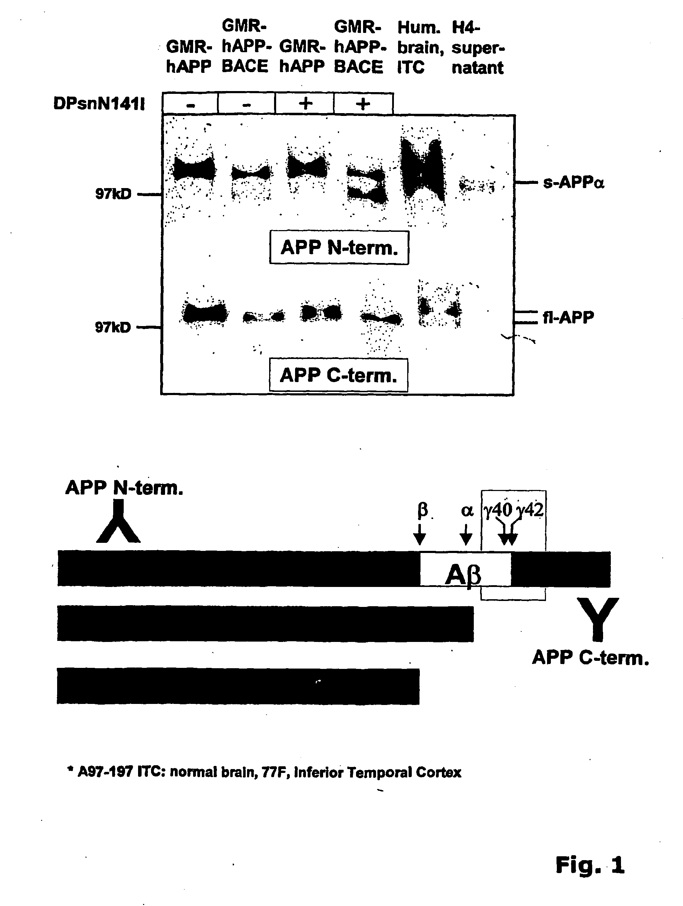

Fig. 1: Processing of hAPP protein in transgenic flies.
   Lane 1 and 2; homogenates of flies expressing the hAPP protein only (1) or in combination with hBACE (2) in all cells of the adult eye (GMR-Gal4). Lane 3 and 4; co-expression of the UAS-DPsnN141l mutant in APP or APP/BACE expressing flies results in an increased processing of the full-length form of APP. Lane 5; homogenate of the inferior temporal cortex region of the brain of a control patient, 77 years, female. Lane 6; the cell culture supernatant of H4 neuroglioma cells expressing the hAPP₆₉₅ gene contains both the sAPPα and sAPPβ secreted forms of hAPP. Bottom diagram; hAPP is a type I transmembrane protein consisting of a large extracellular N-terminal domain, a single transmembrane region and a short cytplasmic tail. α and β-secretase cleavage generates two secreted forms sAPPα and β. β and γ-secretase processing releases the toxic β-amyloid peptide whereas α-secretase cleavage is non-amyloidogenic.

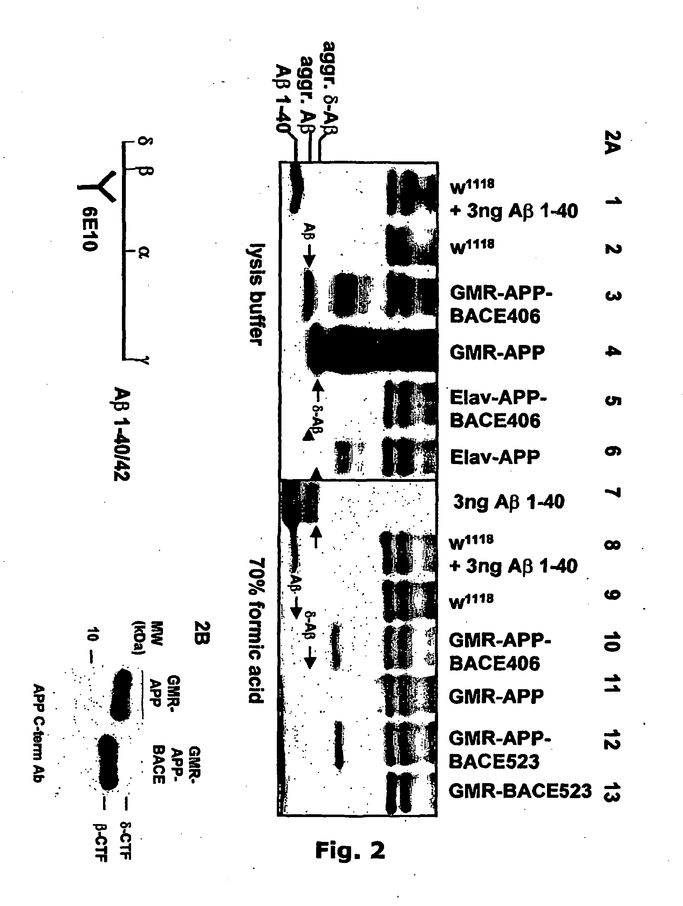

Fig. 2: Generation of Aβ peptides in transgenic flies. A; Immunoprecipitation of Aβ using the monoclonal antibody 6E10 directed against the N-terminus of Aβ. Flies were homogenized either in lysis buffer (lane 1-6) or in 70% formic acid (lane 7-13) prior to precipitation. Lane 2 and 9; homogenate of W¹¹¹⁸ flies representing the genetic background used for generating the BACE transgenic flies and serving as a negative control in immunoprecipitations. Lane 1 and 8 same as lane 2 and 9 except 3ng synthetic Aβ peptide was added to the fly homogenate prior to immunoprecipitation. Lane 7; 3ng synthetic Aβ peptide showing both the 4 kD monomeric and 5 kD aggregated form of Aβ. Lane 3, 10 and 12 Expression of hAPP and hBACE in the adult eye of *Drosophila* using the GMR-Gal4 line and two independent UAS-hBACE transgenes (BACE406, lane 3 and 10; BACE523 lane 12). Detection of the aggregated (lane 3) and monomeric form of Aβ (lane10, 12). The BACE523 expressing line generates slightly more Aβ (compare lane 10 and 12). Lane 13; In flies expressing the hBACE gene only no Aβ peptide are detectable. Lane 4 and 11; flies expressing the hAPP gene generate a 6 kD δ-Aβ peptide with similar aggregation behavior as seen with Aβ. B; Detection of β-and δ-C-terminal fragments (CTF) in APP and APP/BACE expressing flies.

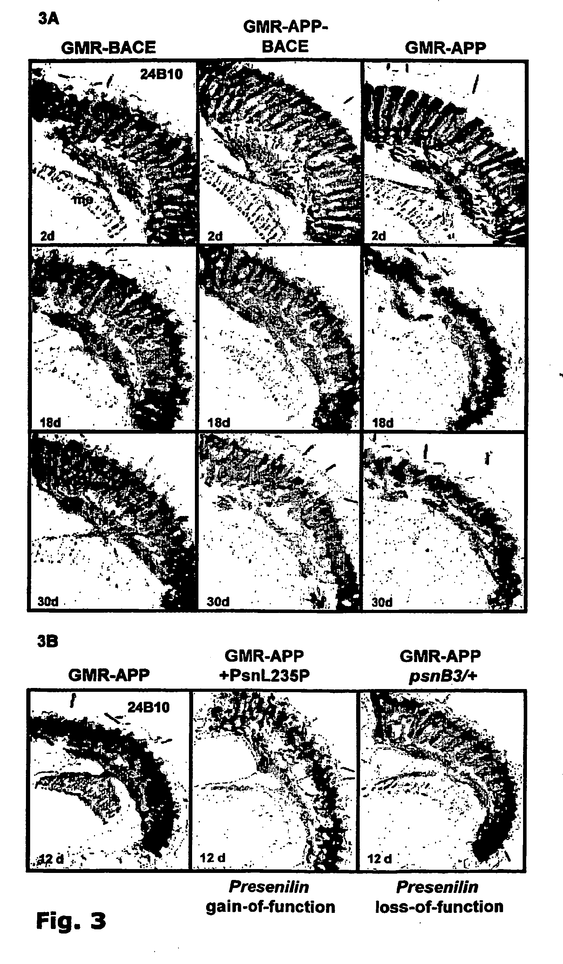

Fig. 3: Age-dependent neurodegeneration in flies expressing the AD associated transgenes under the control of the eye-specific gmr-GAL4 line. A; Staining of photoreceptor cells in the retina (re) and their axonal projections to the lamina (la) and the medulla (me) on cryostat sections of different transgenic fly lines at day 2, day 18 and day 30. Photoreceptor degeneration in *w; UAS-APP*/+; *gmr-GAL4*/+ and w; *UAS-APP*/+; *gmr-GAL4*/*UAS-BACE* transgenic flies but not in *w; gmr-GAL4*/*UAS-BACE* transgenic control flies depends on age. The observed age-dependent degeneration of photoreceptor cells was not gender specific. B; Photoreceptor staining of transgenic flies at day 12. Loss-of *presenilin* function in *w; UAS-APP*/+*; gmr-GAL4*/*psnB3* flies rescues the neurodegeneration, gain of *presenilin* function in w; *UAS-APP*/+*; gmr-GAL4*/*UASDPsnL235P* transgenic flies aggravates the phenotype. Scale bar in (A) 45 µm, in (B) 50µm.

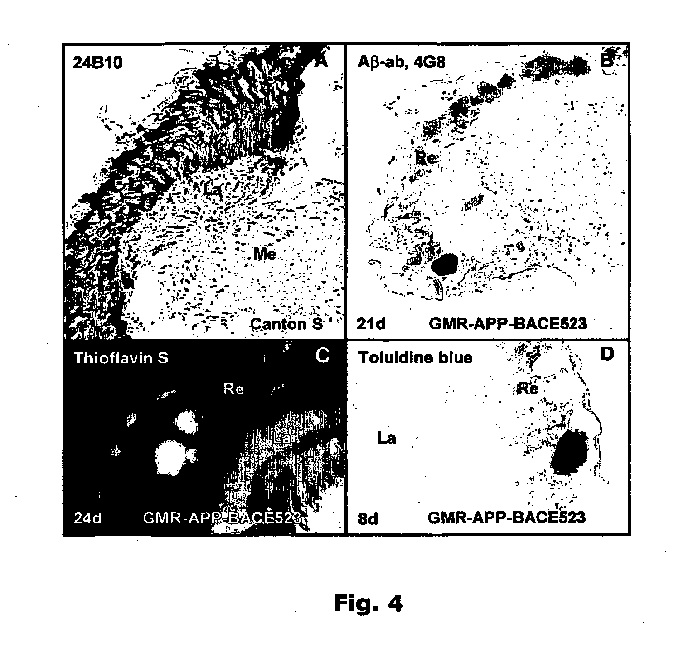

Fig. 4: Aβ deposits in the retina of flies expressing the hAPP and hBACE gene. A; Wild-type optic lobes stained with a photoreceptor cell specific antibody 24B10. B; 4G8 positive plaques on 10µm frozen horizontal brain sections of 21d old flies. The antibody is directed against amino acids 17-24 of Aβ and is frequently used to characterize amyloid-plaques on mammalian brain sections. C; frontal view of a brain section. Thioflavin S positive plaques are stained in the retina of 24d old flies. D; Toulidine blue stains deposits on tangential plastic sections through the adult eye. Note the severe degeneration of the retina in all three panels. Re, retina; La, lamina; Me, medulla.

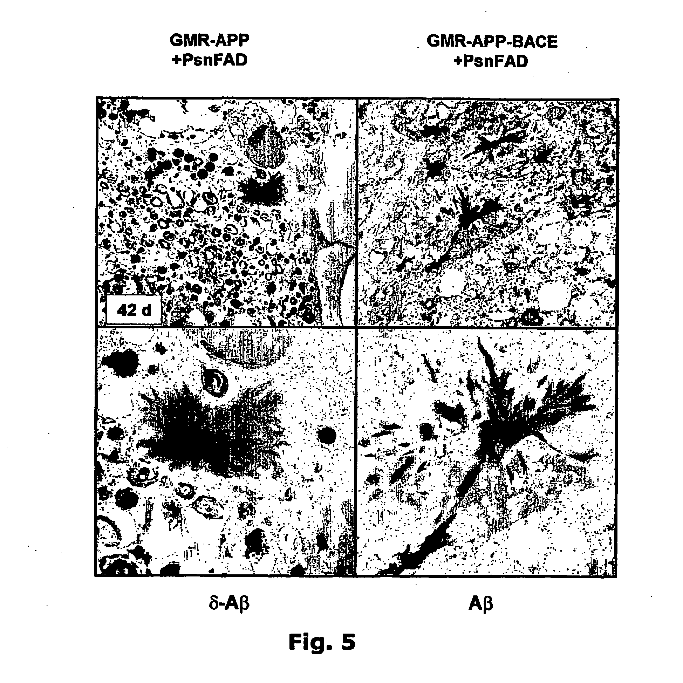

Fig. 5: Ultrastructure of amyloid deposits in electron micrographs of the retina of *w; UAS-APP*/+*; UAS-DPsnL235P*/ *gmr-GAL4* transgenic flies (E, G) and in neuropil regions of the optic lobes in w; *UAS-APP*/*UAS-BACE; UAS-DPsnL235P*/*gmr-GAL4* transgenic flies. Scale bar 9 µm.

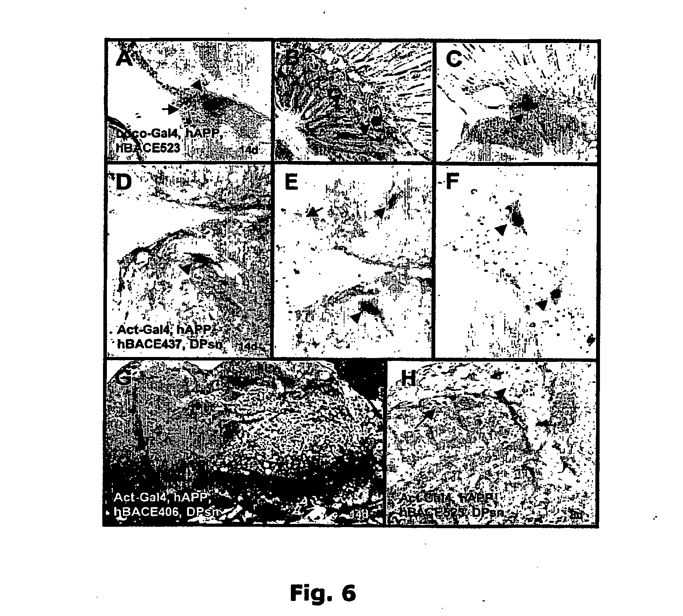

Fig. 6: β-Amyloid-like plaques formation and neurodegeneration in fly brains. A-C; Toluidine blue stained 1µm frontal brain sections of 14d old flies co-expressing hAPP and hBACE under the control of Loco-Gal4, a glial cell specific promoter. Brains demonstrate large amyloid-like plaque deposits (arrow heads) and the majority of the glial cells profoundly change their morphology and show a marked swelling of the cell body (arrows). D-H; Toluidine blue stained 1µm frontal brain sections of 14d old flies (D-G) and an 8d old fly (H) co-expressing hAPP, hBACE and an additional copy of the *Drosophila* presenilin gene ubiquitously in all cells. D-F; Co-expression of hBACE437 leads to plaques formation (arrow heads) and vacuolization (arrows) in the cortex. G; Sponge-like degeneration of the brain expressing hBACE406. H; Neuronal swelling (arrow heads) and vacuolization (arrows) in brains co-expressing hBACE523.

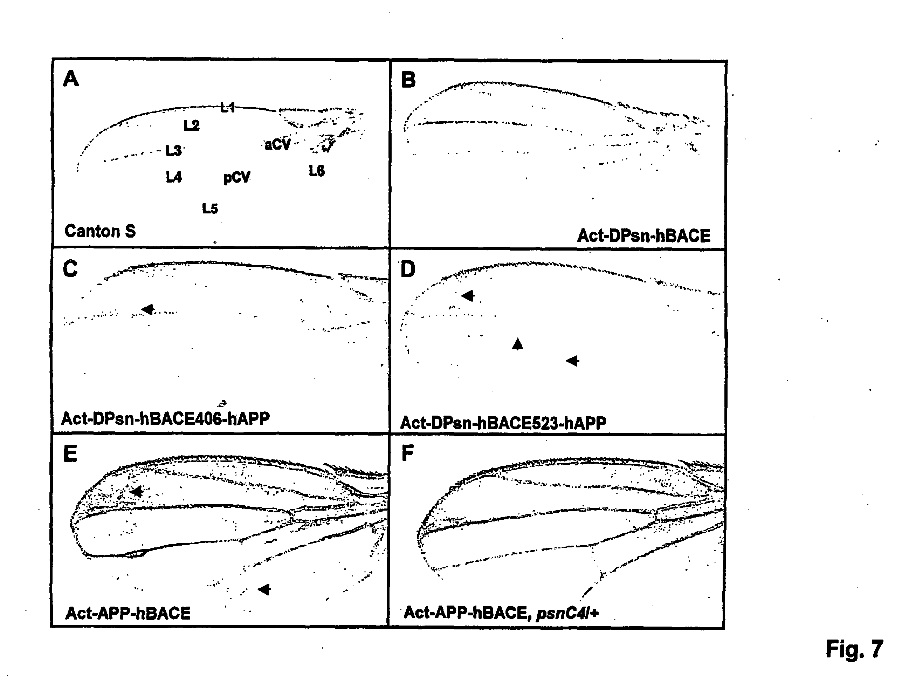

Fig. 7: Defects in wing development.
   A; wild-type Canton S fly showing the wing vein pattern of longitudinal veins (L1-L6) and anterior as well as posterior cross veins (aCV, pCV). B; Expression of DPsn and hBACE does not alter the vein pattern or shape of the wing. C,D,E; β- and γ-secretase processing of hAPP causes ectopic wing vein formation. F. Introducing a loss-of-function *presenilin* allele (*psn*C4/+) rescued the wing vein phenotype almost completely.

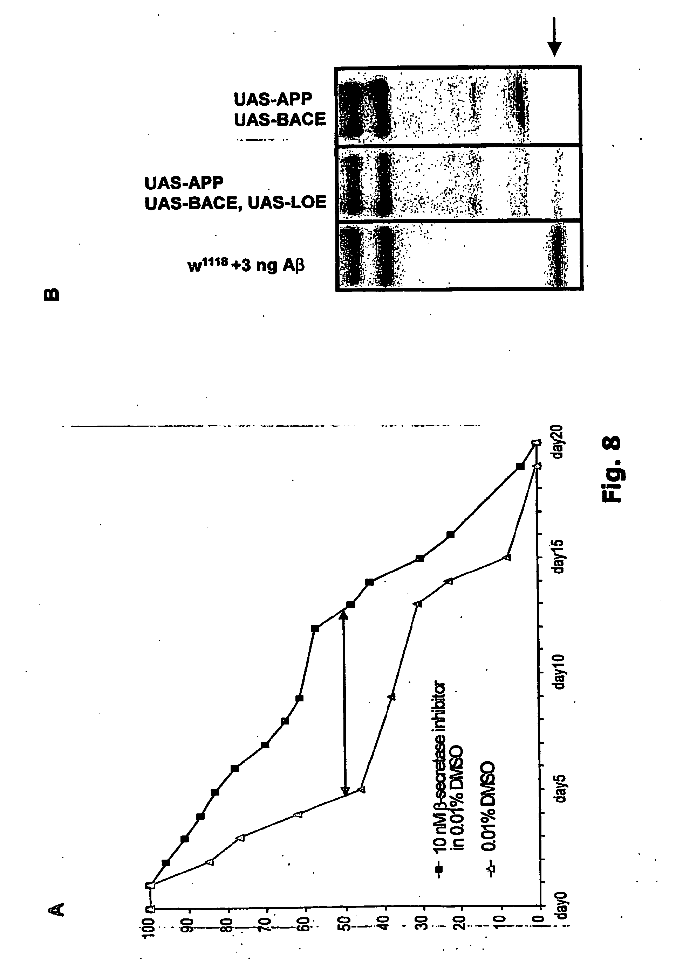

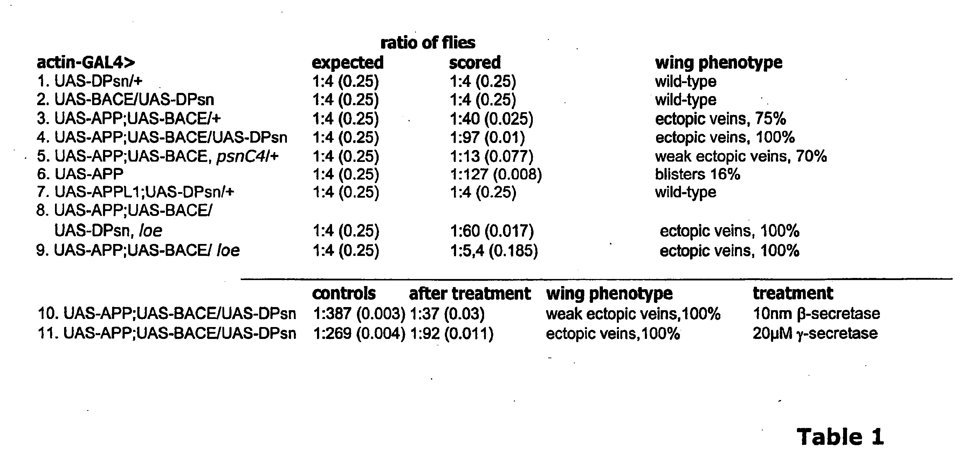

Fig. 8: Increased median life-span of BACE-inhibitor treated flies and elevated levels of Aβ in flies overexpressing *löchrig* in the adult compound eye. A; Survival curve of *w; act-GAL4*/*UAS-APP;UAS-BACE*/*UAS-DPsn* transgenic adult flies after prolonged treatment with the BACE-inhibitor (black squares) and of non treated control flies (grey triangles). B; Immunoprecipitation and detection of Aβ using mab 6E10 from *w; gmr-GAL4*/ *UAS-BACE; UAS-APP*/*UAS-Loe* transgenic flies in comparison to *w; gmr-GAL4*/*UAS-BACE; UAS-APP*/+ transgenic flies.
Fig. 9: Staining of the central and peripheral nervous system of *Drosophila* embryos at the end of embryogenesis using a monoclonal antibody directed against the ELAV protein of *Drosophila.* Left panel: embryos mutant for the *Drosophila* presenilin gene (picture taken from Ye and Fortini, J Cell Biol 1999, 146:1351-1364). Right panel: w¹¹¹⁸ embryos treated with the γ-secretase inhibitor DFK-167 at a concentration of 100µM. Note the severe hyperplasia of cells of the central and peripheral nervous system in both cases due to altered notch-processing.

Table 1: Semi-lethality and wing phenotypes of flies ubiquitously expressing APP, BACE and DPsn as a model system to identify genetic modifiers and validate pharmacologically active agents. Ratios of flies expressing the indicated transgenes under the control of the actin-GAL4 driver to non-expressing flies. A comparison of expected and scored ratios indicates increased lethality during development of the fly. Lethality rate and wing phenotypes of transgenic flies (lines 1-9) and of control treated versus BACE-or γ-secretase inhibitor treated transgenic flies (lines 10 and 11) are shown.

### Example 1

Processing of hAPP (human β-amyloid-precursor protein) in transgenic flies: The hAPP protein is cleaved by three different proteinases termed α, β and γ secretases (Fig.1 schematics). β and γ secretase-type processing releases a small 40-42 amino acid β-amyloid (Aβ) peptide. The extracellular deposition of the toxic insoluble form of the peptide in β-amyloid plaques is one hallmark of the Alzheimer's Disease (AD) pathology and is discussed as the leading cause for the progressive neurodegeneration seen in AD patients.

The entire open reading frame of the previously published human β-secretase gene (β-site APP cleaving enzyme, BACE; Vassar et al., Science 1999, 286:735-741) was cloned into the plasmid pUAST, and 18 independent transgenic *Drosophila* lines (UAS-BACE) were established by germline transformation (Rubin and Spradling, 1982; Spradling and Rubin, 1982). Three different P-element lines were used for the experiments. The *Drosophila* homologue of the human γ-secretase (*Drosophila* presenilin, DPsn) is highly conserved and was shown to process hAPP at the γ-secretase site in APP transgenic flies (Ye and Fortini, J Cell Biol 1999, 146:1351-1364). Transgenic flies expressing the human APP695 gene (UAS-APP) and four fly lines expressing *Drosophila presenilin* (UAS-DPsn) wild-type and point mutations that correspond to the FAD mutations N1411, L235P, E280A were published previously (Fossgreen et al., Proc Natl Acad Sci USA 1998, 95:13703, Ye and Fortini, 1999, see above). Cell specific and ubiquitous expression of the above mentioned transgenes were achieved by using the Gal4/UAS system (Brand and Perrimon, 1993).

The human β-site APP cleaving enzyme (hBACE) processes the human APP protein effectively at the β-secretase site when the two genes are coexpressed in the *Drosophila* compound eye using the GMR-Gal4 line (Fig.1). Western blots of APP in UAS-APP expressing flies with antibodies against the N- or C-termini detected two differently glycosylated full-length forms of APP that comigrated at ∼100kDa with glycosylated APP695 from human brain tissue (Fig. 1, lanes 1, 3, 5). Transgenic co-expression of human BACE with APP resulted in the loss of the higher glycosylated APP together with the generation of a smaller, 97kDa N-terminal fragment that corresponded in molecular mass to the β-cleaved secreted ectodomain of APP (APPsβ) (Fig. 1, lanes 2 and 4). Thus, in *Drosophila,* human BACE appeared to cleave preferentially the higher glycosylated form of APP. Co-expression of each of the UAS-DPsn FAD mutants in UAS-APP/UAS-BACE double transgenic flies consistently increased the processing of full-length glycosylated APP to APPsβ (Fig. 1A, upper panel, compare lane 2 and 4). These data suggest that β-and γ-cleavage of APP were tightly regulated in the *Drosophila* lines of the instant invention.

### Generation of β-amyloid in transgenic flies:

To verify whether hAPP/hBACE transgenic flies generate the toxic insoluble form of Aβ immunoprecipitations were performed using the monoclonal antibody 6E10 under two conditions (Fig. 2). The antibody recognizes an epitope within the first 16 amino acids (aa) of Aβ (Fig. 2 schematics). Flies were homogenized either in lysis buffer or in 70% formic acid before precipitation of endogenous Aβ. Under lysis buffer conditions the antibody precipitates an aggregated form of Aβ (lane 3, arrow) in flies expressing the human APP and human BACE gene in the adult eye (GMR-Gal4). The aggregated form of Aβ appears 1 kD longer than the monomeric form of 4kD and is also seen in a control lane (7, arrow) loaded with synthetic Aβ. When flies were homogenized in 70% formic acid before precipitation the 5 kD aggregated form of Aβ was reduced to the 4 kD monomeric form (compare lane 3 and 10, arrows). The generation of Aβ in flies expressing the human APP and human BACE gene but not the human γ-secretase (presenilin-1) confirms that the *Drosophila* γ-secretase homologue (D-presenilin) is a functional homologue of the human presenilin-1 gene with respect to the cleavage of hAPP (Fossgreen et al., 1998, see above).

Interestingly, flies expressing only the human APP gene generate a 6 kD peptide which can be precipitated using the Aβ specific antibody 6E10 (GMR-APP, lane 4). The alternatively cleaved Aβ peptide also exists in an aggregated and monomeric form under lysis buffer and 70% formic acid conditions (compare lane 4 and 11, arrows). The *Drosophila* genome project does not reveal a *Drosophila* homologue to the human BACE gene. Therefore, we predict that an as yet unidentified endogenous *Drosophila* enzyme cleaves the hAPP protein 10 to 20 amino acids N-terminal to the known β-secretase site and generates a 6kD peptide containing the entire Aβ region at the C-terminus. We named the as yet unknown *Drosophila* enzyme δ-secretase and the alternatively cleaved 6kD peptide, δ-Aβ.
To characterize δ-secretase processing of APP in *Drosophila* in the absence of BACE, we immunoprecipitated the C-terminus of APP with mab 6E10, and subsequently used a polyclonal antibody raised against the last 20 C-terminal amino acids for detection on Western blots (Selkoe et al., Proc Natl Acad Sci USA 1988, 85:7341-5). In UAS-APP flies, 6E10 precipitated an APP C-terminal fragment with an apparent molecular mass of 13 kDa, as compared to the expected 11kDa C-terminal fragment in UAS-APP/UASBACE expressing flies (Fig. 2B).
In addition, several Gal4 and BACE-transgenic lines were compared for the amount of Aβ generated. For example, Elav-Gal4 expresses the transgenes in all neurons of the peripheral and central nervous system of *Drosophila.* The amount of Aβ peptides generated under these conditions is considerable less when compared to flies expressing the transgenes in all cells (photoreceptor cells, pigment cells and cone cells) of the compound eye of *Drosophila* (compare lane 3 and 4 with lane 5 and 6). We therefore initially used the GMR-Gal4 line to express the human hAPP and hBACE gene and looked for phenotypic consequences in immuno-histochemical stainings of sections through the optic system of *Drosophila* (Fig. 3).

### Phenotypic analysis:

Age dependent degeneration of the retina in transgenic flies: The adult eye of *Drosophila* consists of approximately 800 unit eyes (ommatidia) each containing 19 cells (8 photoreceptors R1-R8, 6 pigment cells, 4 cone cells and 1 bristle cell). The adult structure develops from a monolayer epithelium called the eye imaginal disc. Development of photoreceptors and accessory cells starts midway through the last larval stage and proceeds through the four stages of pupal development. The GMR-Gal4 line expresses the transcriptional activator Gal4 in all cells of the compound eye from the larval stage on until adult hood and throughout adult life.

When expressing the hAPP and hBACE gene or the combination of both genes under the control of GMR-Gal4 in the developing eye, no phenotypes are detectable on horizontal sections through the adult optic lobes and retina in 1-2 d old flies (Fig. 3A). The sections were stained with a monoclonal antibody 24B10 recognizing an epitope on the surface of photoreceptor cell bodies and their axonal projections into the first and second optic ganglia (la; lamina and me; medulla, Fig. 3A). This suggests that the development and patterning processes of photoreceptor- and accessory cells are not inhibited and path finding as well as target recognition of photoreceptor axons appear to be unaffected.

In contrast, an age-dependent degeneration of the retina is seen in 18d and 30d old flies expressing the hAPP gene and the combination of hBACE and hAPP (Fig 3A, middle and right panel). However, age matched GMR-BACE406 expressing flies (18d, 30d, Fig. 3A, left panel) do not show any signs of retinal degeneration with increasing age. These results indicate that expression of the full length form (GMR-Gal4, UAS-APP) and the β-secretase processed form of hAPP (GMR-Gal4, UAS-APP, UAS-BACE) is toxic only in differentiated post-mitotic neurons and other cells in an age-dependent manner. The full length form of hAPP is processed at an as yet unidentified site 10-20 amino acids proximal to the known β-secretase cleavage site (8-claveage) and hAPP expressing flies generate the 6kD amyloidogenic peptide (δ-Aβ) containing the entire Aβ peptide at the C-terminus. The remarkable similarities of phenotypes seen in hAPP and hAPP/hBACE expressing flies suggest that at least part of the toxic effects may be due to the generation of amyloidogenic peptides in both genotypes.
To demonstrate that the age-dependent neurodegeneration in the flies of the instant invention was linked to amyloidogenic APP processing rather than neurotoxicity of the full length protein,the γ-secretase activity of the flies was modulated by co-expressing the *Drosophila* FAD *presenilin* genes or by introducing loss-of-function alleles of the endogenous *presenilin* gene that reduce γ-secretase processing. *Presenilin* loss-of-function mutations *psnC4, psnK2* or *psnB3,* or one of three different human FAD mutants UAS-DPsnN141l, E280A or L235P (13, 23) were expressed in w; *UAS-APP*/+*; gmr-GAL4*/*+* transgenic background. At 12 days of age, the heterozygous *presenilin* loss of function mutants (*w; UAS-APP*/+*; gmr-GAL4*/*psnB3*) suppressed the degeneration of photoreceptor cells of UAS-APP expressing flies, whereas co-expression of the *presenilin* FAD mutant *L235P* (*w; UASAPP*/+*; gmr-GAL4*/*UAS-DPsnL235P*) enhanced the degeneration (Fig. 3 B).

### β-amyloid plaques in the retina:

The major component of senile plaques and cerebrovascular deposits in Alzheimer dementia is the 40-42 amino acids Aβ-peptide. Aβ neurotoxicity depends on the state of aggregation and fibril formation of the peptide. Increased production and fibril formation of Aβ-peptides is believed to be the basic pathogenic mechanism leading to a particular aggressive form of the disease seen in several dominant, inherited forms of familiar Alzheimer dementia cases (Selkoe, Nature 1999, 399:A23-31).

In an attempt to correlate the neurodegenerative phenotypes observed after expression of hAPP and hBACE with fibril formation of Aβ we were looking for plaque formation in the adult retina of *Drosophila* using three different techniques (Fig. 4). An antibody against amino acids 17-24 of Aβ (mab 4G8) stains large deposits in the retina of GMR-hAPP-hBACE523 expressing flies (Fig. 4A). Unspecific toluidine blue staining of 1µm plastic sections shows a large extracellular deposit with the typical cloudy appearance characteristic of Aβ-plaques (Fig. 4C). The most specific staining of fibrillar Aβ deposits is shown in Fig. 4B. Thioflavin S stains fibrillar Aβ deposits and emits a green/yellow fluorescence signal under a fluorescence microscope. Large thioflavin S positive plaques are observed in the retina of 24 d old flies with this technique. In addition, the degeneration of retina cells is again evident in all three panels.

### Ultrastructural analyses of Aβ and δ-Aβ containing plaques:

In *Drosophila* β-amyloid plaques formed by the deposition of either δ-Aβ or Aβ shared a similar, if not identical ultrastructure (Fig. 5). Electron microscopy of ultra-thin sections detected many "star-like" β-amyloid deposits of various sizes in the retina and neuropil regions of the first and second optic ganglion of flies generating either δ-Aβ *(w; UAS-APP*/+*; GMR-Gal4*/*UAS-DPsnL235P,* Fig. 4 E, G) or Aβ (*w; UAS-APP*/*UAS-BACE437; GMR-GAL4*/*UAS-DPsnL235P,* Fig. 4 F, H). The β-amyloid plaque had a characteristic fibrillar ultrastructure which resembled β-amyloid plaques in brains of human AD patients or APP-transgenic mice.The fact that β-amyloid deposits were also localized outside the retina, despite expression of the transgenes in photoreceptor cells, strongly suggests that photoreceptor cells secreted Aβ into their projection areas, in addition to local secretion within the retina.

Neurodegeneration and amyloid-like plaques in fly brains with strong and ubiquitous expression of the transgenes:
In addition to the GMR-Gal4 line, Loco-Gal4 was used to express hAPP and hBACE in glial cells and Actin-Gal4 was used for expression in neuronal and non-neuronal cells of the fly. The coexpression of hAPP and hBACE under the control of Loco-Gal4 leads to severe degeneration of glial cells. The glial cells profoundly change their morphology and demonstrate a marked swelling of the cytoplasm (Fig. 5A, B, arrows). In addition, large amyloid-like plaques could be detected in proximity to the degenerating glial cells (Fig. 5A, B, C, arrow heads). Ectopic expression of hAPP, hBACE and an additional copy of the *Drosophila presenilin* gene to enhance the processing of hAPP induced severe neurodegeneration with massive vacuolization. The degree of neurodegeneration differs in the various lines depending on the hBACE transgene used (Fig.5 D,E,F; BACE437, G, BACE406; H; BACE523). In the brain of flies co-expressing the BACE437 transgene, large swollen cell bodies and many plaque-like structures are predominant (Fig. 5A-C), whereas in flies co-expressing the BACE406 transgene the whole brain is almost completely vacuolized and appears like a sponge (Fig. 5G). The co-expression of the BACE523 transgene leads to less neurodegeneration at a younger age, but beginning vacuolization and cell shape changes can be detected in the neuropil and the cortex (Fig. 5H).

### Developmental defects in wing formation and viability of transgenic flies:

Apart from the plaque formation and severe neurodegenerative phenotypes described above, ubiquitous expression of hAPP, hBACE and DPsn under the control of Act-Gal4 leads to defects in wing vein formation during development of the wing. The adult wild-type wing contains six longitudinal wing veins (L1-L6) and two anterior and posterior cross veins (aCV, pCV, Fig. 7A). In comparison to wild-type (Fig. 7A) or DPsn/hBACE expressing flies (Fig. 7B), processing of the hAPP protein by β- and γ-secretases promotes ectopic wing vein formation (Fig. 7C, D, E). The hBACE523-transgene enhances the phenotype of Act-DPsn-hBACE406-hAPP presumably due to elevated expression and higher activity of the hBACE constructs in these genotypes depending on the site of integration of the respective transgene in the genome. Therefore, the observed developmental defects in wing vein formation seem to be modulated by the activity of the human β-secretase. In addition, reducing the activity of the endogenous γ-secretase by introducing a *presenilin* loss-of-function allele (Fig. 7F; *psnC4*/+) rescued the ectopic wing vein phenotype of flies expressing APP and BACE (Fig. 7E) almost completely. These data provide a proof of principle for the use of the model system of the instant invention in genetic modifier screens to uncover new genes that are involved in the processing of the transgenic APP protein.

The expression of a hAPP gene only causes a blistered wing phenotype as described previously by Fossgreen et al., 1998, see above). This phenotype could be observed in 20% of the wings scored whereas ectopic wing vein formation after β- and γ-secretase processing of hAPP was observed in 100% of the wings scored. The blistered wing phenotype may be due to the full length form of the protein as suggested by Fossgreen et al. (see above). The authors discuss the possibility that hAPP expression interferes with cell adhesion independent of Aβ formation. The low penetrance of the phenotype in our genetic system could therefore be a consequence of the impartial processing of the full length form at the as yet unidentified cleavage site upstream of the β-secretase site leaving a considerable amount of full length protein unprocessed (Fig. 1, lane 1).

The generation of ectopic wing veins as well as blistered wings may involve the cytoplasmic C-terminus of hAPP. Recently, a function in gene expression was demonstrated for the cytoplasmic tail of hAPP (Cao and Sudhof, Science 2001, 293:115-20). The C-terminus of hAPP after γ-cleavage forms a multimeric complex with the nuclear adapter protein Fe65 and histone acetyltransferase Tip60 and stimulates transcription in transactivation assays in cell culture using Gal4- or LexA-luciferase reporter plasmids. Development of the wing veins depends in part on the function of two genes, *blistered*/*Serf* and *rhomboid. Blistered*/*Serf,* the *Drosophila* homologue of the mammalian serum response factor and *rhomboid* (a transmembrane protein) are expressed in a complementary pattern in inter vein tissue and future vein cells, respectively. *Blistered*/*Serf* seems to play a dual role in the differentiation of wing tissue. The gene codes for a MADS-box-motif transcription factor and the first target appears to be *rhomboid. Rhomboid* expression is restricted to vein tissue through the function of *blistered*/*Serf* in inter vein cells. In mutant *blistered*/*Serf* inter vein tissue *rhomboid* is expressed ectopically and extra veins are formed. Mutant cells for *blistered*/*Serf* tend to grow along veins and differentiate into vein cells. This phenotype is remarkable similar to what is observed after expression of hAPP and hBACE in all cells of the wing pouch (Fig. 6D, E, F). A second target of *blistered*/*Serf* seems to involve genes of the integrin family of *Drosophila. Myospheroid,* a position specific integrin interacts genetically with *blistered*/*Serf* and mutations in *myospheroid* show a blistered wing phenotype due to the loss of cell/cell adhesion between the dorsal and ventral epidermal wing layers (Sturtevant and Bier, Development 1995, 121:785-801; Prout et al., Genetics 1997, 146:275-285; Nussbaumer et al., Mech Dev 2000, 96:27-36).

In the present invention, it is suggested that γ-secretase dependent processing of the C-terminus of hAPP interferes with *blistered*/*Serf* function during patterning and differentiation of vein and inter vein tissue of the wing. This case provides the first genetic model for studying the signaling function of the C-terminal fragment of hAPP. The sequence of secretase processing starts with the cleavage of APP at the α- or β-secretase site located outside the transmembrane region of hAPP followed by γ-secretase processing within the transmembrane region. A pre-processing of APP by α or β-secretase is absolutely required for γ-secretase processing. In flies expressing the hAPP gene only, the full length form of APP is processed close to the known β-secretase site but to a lower degree when compared to flies co-expressing hAPP and hBACE (Fig. 1). This difference in pre-processing of hAPP probably leads to variable amounts of γ-secretase processed C-terminal fragments in both genetic systems and may explain the differences of phenotypes observed in hAPP expressing flies versus hAPP/hBACE expressing flies and additionally, when using various hBACE transgenes.

Reduced viability and premature death are another feature of flies co-expressing hAPP, hBACE and DPsn genes (Table 1). The ratio of flies expressing all three constructs under the control of Act-Gal4 to non-expressing (Act-Gal4 minus) flies is greatly reduced and again varies depending on the BACE transgene used. Lethality occurs predominantly during embryonic development. 60% of the flies expressing all three transgenes die as embryos whereas 20% show lethality during larval development and the remaining 20% are pupal lethals. The adult escapers show a reduced life span between 7-20 days.
The present invention intends to exploit the reduced viability and life span of flies expressing hAPP, hBACE and DPsn (Table 1) as a model system which has utility for testing the function of candidate β- and γ-secretase inhibitors *in vivo* and for identifying genetic modifiers. As indicated in Table 1 viability and wing phenotype vary dependingon the presence or absence of a *Drosophila presenilln* transgene (DPsn, compare lane 3/4/5). Replacing the human APP by its *Drosophila* homologue APPL which does not contain an Aβ domain completely rescued the lethality (Table 1, line 7). We expected to rescue the larval and pupal lethality and low life span through feeding the respective inhibitors to flies at the larval stage simply by adding the inhibitor to the fly food. Consequently, in order to determine whether these phenotypes are suitable for the validation of therapeutic drugs designed to reduce Aβ formation, either BACE inhibitors or γ-secretase inhibitors were added to the food of transgenic larvae. The eclosion rates of inhibitor treated and nontreated control larvae that were raised on medium supplemented with the inhibitor diluting agent were evaluated. Treatment of *UAS-APP*/*UAS-BACE*/*UAS-DPsn* larvae with the BACE inhibitor at concentrations ranging from 1nM to 50 nM increased the survival rates in a dose dependent manner with a maximum effectiveness at 10 nM. Whereas 1nM BACE-inhibitor had no effect on the survival rate of transgenic flies, 5nM and 10nM BACE-inhibitor increased the survival rate by a factor of 2 and 10, respectively (Table 1, line 11). In addition, the BACE-inhibitor reduced the ectopic wing vein phenotype underscoring that it was related to aberrant APP processing during development. Treatment with the γ-secretase inhibitor at a concentration of 20µM almost tripled the survival rates (0,004 versus 0,011) (Table 1, line 11). Prolonged treatment of the adult flies with the BACE-inhibitor markedly increased their median life span from 5 days to 13 days in comparison to the control flies (Fig. 8 A).
Together, these data encouraged us to employ the *Drosophila* model of the present invention for the identification of genes involved in the regulation of APP processing in the fly. As an example, the novel neurodegeneration mutant *löchrig* (Tschäpe et al., 2001, abstract of papers presented at the 2001 meeting on the neurobiology of *Drosophila,* Cold Spring Harbor, New York)) was tested on the survival rates in the actin-GAL4 overexpression background. The mutant *löchrig* encodes a novel isoform of the γ-subunit of AMP-activated protein kinase which regulates the activities of HMG-CoA reductase and hormone-sensitive lipase. The *löchrig* mutants have low levels of cholesterol esters along with severe and age-dependent degeneration of the central nervous system. Cholesterol levels influence APP processing, and intracellular cholesterol esters affect Aβ generation. In the *Drosophila* lines of the present invention, the inactivation of one copy of the endogenous *löchrig* gene rescued the lethality of w; *actin-GAL4*/*UAS-APP; UAS-BACE*/*UAS-DPsn* (Table 1, line 4) and *w; actin-GAL4*/*UAS-APP; UAS-BACE*/+ flies 1.6 fold and 7.4 fold, respectively (compare Table 1, line 4 with 8 and line 3 with 9). To demonstrate a more direct role for *löchrig* in APP processing and Aβ generation, *löchrig* was overexpressed along with APP and BACE in the adult eye, and Aβ levels were compared from UAS-BACE/UAS-APP/UAS-Loe expressing flies with UAS-BACE/UAS-APP flies (Fig. 8 B). Co-expression of UAS-Loe caused a marked increase in SDS-soluble Aβ detected on Western blots (arrow in Fig 8 B) and led to earlier depositions of Aβ in thioflavin S positive β-amyloid plaques in the retinas. These findings indicate that the *Drosophila* model of the present invention is applicative for identifying and analyzing genes that can modulate Aβ generation and amyloid plaque formation.

### Validation of β- and γ-secretase inhibitors using wild-type flies:

The cloning and characterization of the human β-secretase and the growing evidence for presenilin-1 acting either in a complex with or representing the γ-secretase led to the generation of peptide analogs serving as inhibitors of the respective enzyme to prevent β-amyloid formation. Presenilin-1 function is not restricted to APP processing but instead is also involved in proteolysis of the notch-receptor (De Strooper et al., Nature 1999, 398:518-522; Struhl and Greenwald, Nature 1999, 398:522-525). Mutations in presenilin-1 and notch in vertebrates and *Drosophila* cause similar embryonic lethal phenotypes including a severe neural hyperplasia during development of the embryonic nervous system (Fig. 9). Therefore, inhibition of γ-secretase function to prevent β-amyloid formation may show toxic side effects through inhibition of notch-receptor processing and nuclear translocation of the C-terminus. The development of the *Drosophila* embryonic nervous system may serve as a model system to test chemical compounds for inhibition of notch signaling. We performed an *in vivo* experiment to test the γ-secretase inhibitor DFK-167 (Sanbio Enzyme Systems). w¹¹¹⁸ embryos were incubated 1.5 hours after egg laying either in 100µM DFK-167 in 0.1% DMSO or in 0.1% DMSO for 1.5 hours and were stained at the end of embryogenesis with an antibody specific for the peripheral and central nervous system. 1% of the embryos treated with the γ-secretase inhibitor at a concentration shown to reduce β-amyloid formation to almost baseline levels in cell culture systems (Wolfe et al., Biochemistry 1999, 38:11223-30; and Biochemistry 1999, 38:4720-7) demonstrated a severe neurogenic phenotype (Fig. 7). This phenotype is identical to a previously described neurogenic phenotype seen in notch and presenilin "loss-of-function" mutants (Fig. 7, De Strooper et al., 1999; Struhl and Greenwald, 1999; Ye and Fortini, 1999). The minor phenotypic consequences of the γ-secretase inhibitor DFK-167 treatment on the processing of the notch-receptor may reflect a relative specificity of the inhibitor for hAPP processing at the indicated concentration. But, nevertheless, it shows that a simple treatment of *Drosophila* embryos may allow a first characterization of chemical compounds with γ-secretase inhibitory function for possible toxic effects *in vivo.*

### Materials and Methods

### Transgenic flies:

BACE transgenic flies: A 1,9kb KpnI-XhoI restriction fragment of the human BACE (beta site APP cleaving enzyme, Vassar et al., Science 1999, 286:735-41) cDNA containing the entire open reading frame was cloned into plasmid pUAST (Brand and Perrimon, Development 1993, 118:401-15). P-element-mediated germline transformation was performed as described by Spradling and Rubin (Rubin and Spradling, Science 1982, 218:348-53; Spradling and Rubin, Science 1982, 218:341-47).
APP and presenilin transgenic flies: UAS-APP695II, UAS-APP695III (Wt-34, Wt-35) and the UAS-Dpsn+14 as well as UAS-Dpsn-mutants (M146V; N141I; L235P; E280A) (Fossgreen et al., Proc Natl Acad Sci USA 1998, 95:13703-8; Ye and Fortini, J Cell Biol 1999, 146:1351-64).

### Fly Genetics:

Flies of the following genotyps were used for setting up crosses: w; Actin-Gal4/CyO - w; Actin-Gal4/CyO; UAS-DPsn/Tm3 - w; UAS-DPsn/Tm3 - w; UAS-BACE406, 437; 523 - w; UAS-hAPP - w; UAS-BACE437; UAS-APP - w; UAS-APP; UAS-BACE406, 523 - w; GMR-Gal4/Tm3 - w; UAS-APP/CyO; GMR-Gal4/Tm3 - w; Loco-Gal4/CyO.

### Protein analysis:

For Western blotting, fly heads were homogenized in 1xPBS, 5mM EDTA, 0.5% Triton X-100 and a protease-inhibitor mix Complete (Boehringer Mannheim) with a dounce homogenizer. Brain tissues were homogenized with mortar and pestle in liquid nitrogen and subsequently with a dounce homogenizer in 20% glycerol, 2% SDS, 125mM NaCl, 0.075M DTT, 1% Triton X-100. H4-Cell supernatants were concentrated and desalted on Centricon YM-50 columns (Amicon). Protein concentrations were measured with a Bradford assay. Equal amounts of protein were separated by 10% SDS-PAGE, transferred to Immobilon membranes (Millipore GmbH), blocked in 5% low fat milk for two hours at room temperature, incubated with the monoclonal Antibody 22C11 against the N-terminal part of APP overnight at 4°C, followed by one hour incubation with the goat anti-mouse peroxidase (Dianova) conjugated secondary antibody at room temperature.

For immunoprecipitation, fly heads were homogenized as described above. Equal amounts of protein were preincubated with 80 µl Protein LA-Agarose (Sigma) for 1 hour at 4°C. The supernatant was transferred and incubated with 100 µl Protein LA-Agarose and 3 µg of the monoclonal antibody 6E10 (QCB Biosource international) against amino-acids 1-16 of the amyloid β peptide overnight at 4°C on a rotating wheel. After 5 times washing of the beads, pellets were resuspended in 2 x SDS sample buffer and analyzed on 10-20% gradient Novex Tris-Tricine Gels (Invitrogen). Western blots for the detection of β-amyloid was performed as described (Ida et al., J Biol Chem 1996, 271:22908-14) using the mAb 6E10.

### Immunohistochemistry and histology:

For immunostaining, adult flies were fixed in 4% paraformaldehyde for 3 hours, washed in 1 x PBS and transferred to 25% sucrose for an overnight incubation at 4°C. Flies were decapitated with a razor blade, heads were imbedded in Tissue Tek (Sakura) and snap frozen on dry ice. Ten µm thick horizontal frozen sections were prepared on a cryostat. Immunostaining was done with the Vectastain Elite kit (Vector Laboratories) according to the manufacturers instructions. Protocol was slightly modified for fluorescent staining. The incubation with the biotinylated secondary antibody is followed by a further incubation for 30 minutes with a Streptavidin-HRP conjugated complex. Finally, slides were incubated with tyramide fluorescein (TSA - Tyramide Signal Amplification System- Fluorescein, NEN) for 10 minutes mounted in Vectashield mounting medium (Vector Laboratories) and analyzed by fluorescence microscopy. The following primary antibodies were used: 24B10 (1:5) and rat anti-ELAV monoclonal antibody 7E8A10 (1:50), both provided by the Developmental Studies Hybridoma Bank, and a mouse monoclonal antibody against amino-acids 17-24 of the amyloid β peptide, 4G8 (1:1000, Signet).

For thioflavin S staining, flies were fixed in 4% paraformaldehyde, washed in 1 x PBS and dehydrated sequentially in 50%, 70%, two times 95%, three times 100% ethanol for 30 mintues each. Flies were decapitated with a razor blade and immersed for 30 minutes in three changes of xylene. Heads were left for 48hrs in paraffin at 63°C (three changes of fresh paraffin during that time). Heads were embedded in paraffin blocks. Horizontal paraffin sections (7µm) were prepared on a microtome and mounted on slides. Paraffin slides were dried for 24 hours at room temperature. Slides were subsequentially deparaffinized in two changes of xylene for 5 minutes and hydrated sequentially in 100%, 96%, 80%, 60% ethanol for 5 minutes each and rinsed in destilled water. Slides were counterstained for 5 minutes in Mayers Hemalum (Sigma), rinsed for 10 minutes in tap water and stained for 3 minutes in 1% thioflavin S (Sigma) aquaeous solution. Slides were rinsed in several changes of distilled water, incubated for 15 minutes in 1% acetic acid, rinsed in tap water and mounted in Vectashield mounting medium (Vector laboratories). Slides were analyzed under fluorescence microscopy (430 nm excitation, 550 nm emission).

For plastic sections, flies were fixed overnight in 5% glutaraldehyde at 4°C, decapitated with a razor blade, washed three times in PBS (5 min each) and treated with 2% osmium-tetraoxid on ice. Sections were again washed three times for 10 min each, sequentially dehydrated in 50%, 70%, 80%, 95%, 3x 100% ethanol for 10 minutes each, incubated in 100% ethanol : epon 1:1 overnight and embedded in epon. Plastic sections (1µm) were cut on a microtome and stained with 1% toluidine blue, 1% borax. Adult fly wings were removed and mounted in DPX mountant (Fluka) for light microscopy.

### γ- secretase inhibitor treatment of wild-type flies:

W¹¹¹⁸ embryos were decorinated 1.5 hours after egg laying and treated with 0.1% DMSO with or without 100µM γ-secretase inhibitor DFK-167 (Sanbio Enzyme Systems) for 1.5 hours. Embryos were rinsed in PBS and allowed to continue to develop until the end of embryogenesis. The embryonic nervous system was stained with rat anti-ELAV as described by Doe (Development 1992, 116:855-63).

### β- and γ-secretase inhibitor treatment of transgenic flies:

For treatment, the β-secretase inhibitor (Calbiochem, no 171601) and the γ-secretase inhibitor (Calbiochem, no 565750) were used. The β-secretase inhibitor was added to the standard fly medium to a final concentrations of 10nM in 0.01%, DMSO; for the γ-secretase inhibitor a final concentration of 20µM in 0.025% acetic acid was used. Flies of the following genotypes were mated: *w; actin-Gal4*/*Cyo; UAS- DPsn*/*Tm3* x *w; UAS-APP*/*UASAPP; UAS-BACE523*/*UAS-BACE523* and eggs were developed on fly medium supplemented with the inhibitors or with the diluting agents (DMSO/acetic acid) through the larval stage until eclosion. The survival rate and extra vein phenotype of flies transgenic for *w; actin-GAL4*/*UAS-APP; UAS-BACE523*/*UAS-DPsn* were scored. Adult transgenic flies were continuously treated at 29°C with or without 10nM β-secretase inhibitor in 0.01% DMSO.

## Claims

1. An insect, preferably a fly that has been modified to express a set of genes, said set comprising a gene coding for a vertebrate amyloid precursor protein (APP), or a fragment, or derivative, or variant thereof, and a gene coding for a vertebrate β-secretase (β-site APP cleaving enzyme).

2. The insect according to claim 1 wherein said insect expresses a gene coding for a protein having γ-secretase activity.

3. The insect according to claim 2 wherein said gene is presenilin.

4. The insect according to claim 2 wherein said gene is endogenous presenilin, or a fragment, or a derivative, or a variant thereof.

5. The insect according to claims 2 to 4 wherein said gene is over-expressed or mis-expressed.

6. The insect according to claims 1 to 5 being transgenic for said genes.

7. The insect according to claims 1 to 6 wherein the expression of said genes results in an identifiable phenotype.

8. The insect according to claims 1 to 7 wherein said identifiable phenotype comprises β-amyloid generation.

9. The insect according to claims 1 to 7 wherein said identifiable phenotype comprises plaque deposition.

10. The insect according to claims 1 to 7 wherein said identifiable phenotype comprises neurodegeneration.

11. The insect according to claims 1 to 7 wherein said identifiable phenotype comprises degeneration of photoreceptor cells.

12. The insect according to claims 1 to 7 wherein said identifiable phenotype comprises degeneration of glia cells.

13. The insect according to claims 1 to 7 wherein said identifiable phenotype comprises reduced viability.

14. The insect according to claims 1 to 7 wherein said identifiable phenotype comprises a wing phenotype.

15. Use of the insect according to any of claims 1 to 14 as an animal model for testing and screening compounds that modulate APP processing.

16. Use of the insect according to any of claims 1 to 14 as an animal model for testing and screening compounds that modulate β-secretase.

17. Use of the insect according to any of claims 1 to 14 as an animal model for testing and screening compounds that modulate γ-secretase.

18. Use of the insect according to any of claims 1 to 14 as an animal model for screening of modifier genes that influence APP processing.

19. A method of testing a compound, preferably of screening a plurality of compounds useful for the modulation of APP processing, said method comprising:
(a) providing an insect, preferably a fly that has been modified to express a set of genes, said set comprising a gene coding for a vertebrate amyloid precursor protein (APP), or a fragment, or derivative, or variant thereof, and a gene coding for a vertebrate β-secretase (β-site APP cleaving enzyme), wherein expression of said set of genes results in an identifiable phenotype,
(b) contacting said insect with a compound or a plurality of compounds,
(c) assaying for changes in said identifiable phenotype of said insect of step (a) as compared to an insect of step (a) not having been contacted with said compound or plurality of compounds.

20. A method of screening for modifier genes that influence the processing of APP, said method comprising:
(a) providing an insect, preferably a fly that has been modified to express a set of genes, said set comprising a gene cording for a vertebrate amyloid precursor protein (APP), or a fragment, or derivative, or variant thereof, and a gene coding for a vertebrate β-secretase (β-site APP cleaving enzyme), wherein expression of said set of genes results in an identifiable phenotype,
(b) crossing said insect with an animal having an alteration in a known or candidate gene other than said set of genes, and
(c) screening progeny of said crosses for animals expressing said set of genes and having said alteration in said known or candidate gene other than said set of genes, wherein a modified identifiable phenotype as compared to controls indicates the presence of a modifier gene that influences the processing of APP.

21. The method of claim 20 further comprising identifying a human homolog of the modifier gene.

## Patentansprüche

1. Insekt, vorzugsweise eine Fliege, das so modifiziert ist, dass es eine Gruppe von Genen exprimiert, wobei die Gruppe ein Gen, das für ein Vertebraten-Amyloid-Vorläuferprotein (APP) codiert, oder ein Fragment oder Derivat oder eine Variante davon sowie ein Gen, das für eine Vertebraten-β-Secretase (BACE1; β-site of APP cleaving enzyme) codiert, umfasst.

2. Insekt gemäß Anspruch 1, wobei das Insekt ein Gen exprimiert, das für ein Protein mit γ-Secretase-Aktivität codiert.

3. Insekt gemäß Anspruch 2, wobei es sich bei dem Gen um das Presenilin-Gen handelt.

4. Insekt gemäß Anspruch 2, wobei es sich bei dem Gen um das endogene Presenilin-Gen oder ein Fragment oder Derivat oder eine Variante davon handelt.

5. Insekt gemäß den Ansprüchen 2 bis 4, wobei das Gen überexprimiert oder fehlexprimiert wird.

6. Insekt gemäß den Ansprüchen 1 bis 5, das transgen in Bezug auf die genannten Gene ist.

7. Insekt gemäß den Ansprüchen 1 bis 6, wobei die Expression der Gene zu einem identifizierbaren Phänotyp führt.

8. Insekt gemäß den Ansprüchen 1 bis 7, wobei der identifizierbare Phänotyp die Erzeugung von β-Amyloid umfasst.

9. Insekt gemäß den Ansprüchen 1 bis 7, wobei der identifizierbare Phänotyp die Ablagerung von Amyloidplaques umfasst.

10. Insekt gemäß den Ansprüchen 1 bis 7, wobei der identifizierbare Phänotyp eine Neurodegeneration umfasst.

11. Insekt gemäß den Ansprüchen 1 bis 7, wobei der identifizierbare Phänotyp eine Degeneration von Photorezeptorzellen umfasst.

12. Insekt gemäß den Ansprüchen 1 bis 7, wobei der identifizierbare Phänotyp eine Degeneration von Gliazellen umfasst.

13. Insekt gemäß den Ansprüchen 1 bis 7, wobei der identifizierbare Phänotyp eine reduzierte Lebensfähigkeit umfasst.

14. Insekt gemäß den Ansprüchen 1 bis 7, wobei der identifizierbare Phänotyp einen geflügelten Phänotyp umfasst.

15. Verwendung des Insekts gemäß einem der Ansprüche 1 bis 14 als Tiermodell zum Testen von und Suchen (Screening) nach Verbindungen, die die APP-Prozessierung modulieren.

16. Verwendung des Insekts gemäß einem der Ansprüche 1 bis 14 als Tiermodell zum Testen von und Suchen (Screening) nach Verbindungen, die β-Secretase modulieren.

17. Verwendung des Insekts gemäß einem der Ansprüche 1 bis 14 als Tiermodell zum Testen von und Suchen (Screening) nach Verbindungen, die γ-Secretase modulieren.

18. Verwendung des Insekts gemäß einem der Ansprüche 1 bis 14 als Tiermodell zum Suchen (Screening) nach Modifikatorgenen, die die APP-Prozessierung beeinflussen.

19. Verfahren zum Testen einer Verbindung, vorzugsweise zum Durchmustern einer Vielzahl von Verbindungen, die für die Modulation der APP-Prozessierung geeignet sein könnten, wobei das Verfahren Folgendes umfasst:
(a) Bereitstellen eines Insekts, vorzugsweise einer Fliege, das so modifiziert ist, dass es eine Gruppe von Genen exprimiert, wobei die Gruppe ein Gen, das für ein Vertebraten-Amyloid-Vorläuferprotein (APP) codiert, oder ein Fragment oder Derivat oder eine Variante davon sowie ein Gen, das für eine Vertebraten-β-Secretase (BACE1; β-site of APP cleaving enzyme) codiert, umfasst, wobei die Expression der Gruppe von Genen zu einem identifizierbaren Phänotyp führt;
(b) In-Kontakt-Bringen des Insekts mit einer Verbindung oder einer Vielzahl von Verbindungen;
(c) Bestimmen der Veränderungen in dem identifizierbaren Phänotyp des Insekts von Schritt (a) im Vergleich zu einem Insekt von Schritt (a), das nicht mit der Verbindung oder der Vielzahl von Verbindungen in Kontakt gebracht wurde.

20. Verfahren zum Suchen (Screening) nach Modifikatorgenen, die die Prozessierung von APP beeinflussen, wobei das Verfahren Folgendes umfasst:
(a) Bereitstellen eines Insekts, vorzugsweise einer Fliege, das so modifiziert ist, dass es eine Gruppe von Genen exprimiert, wobei die Gruppe ein Gen, das für ein Vertebraten-Amyloid-Vorläuferprotein (APP) codiert, oder ein Fragment oder Derivat oder eine Variante davon sowie ein Gen, das für eine Vertebraten-β-Secretase (BACE1; β-site of APP cleaving enzyme) codiert, umfasst, wobei die Expression der Gruppe von Genen zu einem identifizierbaren Phänotyp führt;
(b) Kreuzen des Insekts mit einem Tier, das eine Veränderung in einem bekannten oder aussichtsreichen Gen, das nicht zu der Gruppe von Genen gehört, aufweist; und
(c) Durchmustern der Nachkommen aus den Kreuzungen nach Tieren, die die Gruppe von Genen exprimieren und die Veränderung in dem bekannten oder aussichtsreichen Gen, das nicht zu der Gruppe von Genen gehört, aufweisen, wobei ein im Vergleich zu Kontrollen modifizierter identifizierbarer Phänotyp auf die Anwesenheit eines Modifikatorgens, das die Prozessierung von APP beeinflusst, hinweist.

21. Verfahren gemäß Anspruch 20, das weiterhin das Identifizieren eines humanen Homologen des Modifikatorgens umfasst.

## Revendications

1. Insecte, de préférence une mouche, qui a été modifié pour exprimer un ensemble de gènes, ledit ensemble comprenant un gène codant pour une protéine précurseur de l'amyloïde (APP) de vertébré ou un fragment, un dérivé ou un variant de celle-ci, et un gène codant pour une β-sécrétase (enzyme clivant l'APP au niveau du site β) de vertébré.

2. Insecte selon la revendication 1, dans lequel ledit insecte exprime un gène codant pour une protéine ayant une activité γ-sécrétase.

3. Insecte selon la revendication 2, dans lequel ledit gène est celui de la préséniline.

4. Insecte selon la revendication 2, dans lequel ledit gène est celui de la préséniline endogène ou un fragment, un dérivé ou un variant de celle-ci.

5. Insecte selon les revendications 2 à 4, dans lequel ledit gène est surexprimé ou mal exprimé.

6. Insecte selon les revendications 1 à 5, qui est transgénique pour lesdits gènes.

7. Insecte selon les revendications 1 à 6, dans lequel l'expression desdits gènes donne un phénotype identifiable.

8. Insecte selon les revendications 1 à 7, dans lequel ledit phénotype identifiable comprend la production de β-amyloïde.

9. Insecte selon les revendications 1 à 7, dans lequel ledit phénotype identifiable comprend le dépôt de plaques.

10. Insecte selon les revendications 1 à 7, dans lequel ledit phénotype identifiable comprend une neurodégénérescence.

11. Insecte selon les revendications 1 à 7, dans lequel ledit phénotype identifiable comprend la dégénérescence des cellules photoréceptrices.

12. Insecte selon les revendications 1 à 7, dans lequel ledit phénotype identifiable comprend la dégénérescence des cellules gliales.

13. Insecte selon les revendications 1 à 7, dans lequel ledit phénotype identifiable comprend une viabilité réduite.

14. Insecte selon les revendications 1 à 7, dans lequel ledit phénotype identifiable comprend un phénotype d'ailes.

15. Utilisation de l'insecte selon l'une quelconque des revendications 1 à 14 en tant que modèle animal pour évaluer et cribler des composés qui modulent le traitement de l'APP.

16. Utilisation de l'insecte selon l'une quelconque des revendications 1 à 14 en tant que modèle animal pour évaluer et cribler des composés qui modulent la β-sécrétase.

17. Utilisation de l'insecte selon l'une quelconque des revendications 1 à 14 en tant que modèle animal pour évaluer et cribler des composés qui modulent la γ-sécrétase.

18. Utilisation de l'insecte selon l'une quelconque des revendications 1 à 14 en tant que modèle animal pour cribler des gènes modificateurs qui influencent le traitement de l'APP.

19. Procédé d'évaluation d'un composé, de préférence de criblage d'une pluralité de composés utiles pour la modulation du traitement de l'APP, ledit procédé consistant à :
(a) fournir un insecte, de préférence une mouche, qui a été modifié pour exprimer un ensemble de gènes, ledit ensemble comprenant un gène codant pour une protéine précurseur de l'amyloïde (APP) de vertébré ou un fragment, un dérivé ou un variant de celle-ci, et un gène codant pour une β-sécrétase (enzyme clivant l'APP au niveau du site-β) de vertébré, dans lequel l'expression dudit ensemble de gènes donne un phénotype identifiable,
(b) mettre ledit insecte en contact avec un composé ou avec une pluralité de composés,
(c) évaluer les changements dudit phénotype identifiable dudit insecte de l'étape (a) par rapport à un insecte de l'étape (a) qui n'a pas été mis en contact avec ledit composé ou avec ladite pluralité de composés.

20. Procédé de criblage de gènes modificateurs qui influencent le traitement de l'APP, ledit procédé comprenant :
(a) la fourniture d'un insecte, de préférence une mouche, qui a été modifié pour exprimer un ensemble de gènes, ledit ensemble comprenant un gène codant pour une protéine précurseur de l'amyloïde (APP) de vertébré ou un fragment, un dérivé ou un variant de celle-ci, et un gène codant pour une β-sécrétase (enzyme clivant l'APP au niveau du site-β) de vertébré, dans lequel l'expression dudit ensemble de gènes donne un phénotype identifiable,
(b) le croisement dudit insecte avec un insecte présentant une altération au niveau d'un gène connu ou candidat autre que ledit ensemble de gènes, et
(c) le criblage des descendants desdits croisements afin de détecter les insectes exprimant ledit ensemble de gènes et présentant ladite altération au niveau dudit gène connu ou candidat autre que ledit ensemble de gènes, dans lequel un phénotype identifiable modifié par rapport à des témoins indique la présence d'un gène modificateur qui influence le traitement de l'APP.

21. Procédé selon la revendication 20 comprenant en outre l'identification d'un homologue humain du gène modificateur.
